# EUROPEAN PATENT APPLICATION

(11) **EP 4 360 464 A1**
(43) Date of publication of application: **01.05.2024**
(21) Application number: 22204131.1
(22) Date of filing: 27.10.2022
(51) Int. Cl.: A23J 1/00, A23L 27/60, A23L 29/10, A61K 8/04, C12F 3/06, A23D 7/005, A23L 15/00

(54) **PROTEIN COMPOSITION**

(71) Applicant: Duynie Holding B.V., 6541 Nijmegen (NL)
(72) Inventor: LOMMERS, Marcel, 6541 Nijmegen (NL)
(74) Representative: Nederlandsch Octrooibureau

(57) **Abstract**

The invention pertains to a protein composition comprising fat and a protein wherein the fat is encapsulated by at least part of the protein and at least 10 wt% of the protein has a molecular weight of at least 3 kDa, and the amount of protein is at least 5 wt%, based on the total weight of the composition.

## Description

The present invention relates to a protein composition.

Non-functional proteins are generally hydrolyzed to increase the functionality of the proteins. Many authors report to hydrolyze the non-functional proteins to a short length that the protein fractions are soluble in water themselves. RU2428047, RU2663583, CN102965424, CN108441536 and JP19991091445 and MY161177 describe hydrolyzation of different proteins including whey protein, soybean and rapeseed protein to form protein hydrolysates with molecular weights below 4 kDa.

Similar approaches have been reported for Brewer's spent grain. Robertson et al (2011; doi:10.1021/jf202814j) discloses the hydrolyzation of Brewer's spent grain using various enzymes such as Alcalase^{®} and Fluorozyme^{®} to form small protein fraction while at the same time the fibers are still present. Vierra et al (2021; doi:10.1111/jfpp.16638) describes the treatment of degreased Brewer's spent grain with Alcalase^{®} to obtain proteins with functional properties. WO 2021/028509 discloses enzymatic protein hydrolysis of Brewer's spent grain to form proteins with a molecular weight of 2 to 4.5 kDa. The functionality of these small proteins is generally limited and commercially not interesting. Moreover, small peptides generally have a bitter taste.

The objective of the present invention is to provide a novel protein composition.

The invention pertains to a protein composition comprising fat and a protein wherein the fat is encapsulated by at least part of the protein and at least 10 wt% of the protein has a molecular weight of at least 3 kDa and the amount of protein is at least 5 wt%, based on the total weight of the composition. The composition of the invention has significantly better emulsifying properties compared to compositions of fat and coagulated proteins such as proteins in brewer's spent grain. Moreover, the inventors have found that the presence of proteins having a molecular weight of less than 3 kDa generally do not have a good affinity to fat and are not capable of encapsulating the fat. It was observed that the presence of proteins having a molecular weight of less than 3 kDa generally reduces the emulsification properties of the protein composition. While not being bound by theory, the combination of fat and protein in the inventive composition generally leads to an interaction between the fat and the protein so that fat is encapsulated by the protein. The proteins exhibiting (stronger) affinity or interaction with fat will make up the encapsulation layer on the fat, and the proteins showing (lower) affinity with fat will remain in solution or suspension. Hence, this allows for selective encapsulation by proteins that have a good affinity to the fat present, which generally leads to improved emulsification properties of the resulting protein composition. It is also contemplated that the interaction between fat and protein remains upon further processing or use in food or cosmetic products, and in itself has good or even better emulsification properties. As a consequence, the proteins that have lower or no affinity to the fat can be removed using conventional techniques such as centrifugation, decantation and/or (ultra)filtration. In addition, the inventive protein composition may be in emulsion form or be used in emulsions, such emulsions generally have a good stability and an improved storage stability. Due to encapsulation of the fat, the protein compositions of the invention can be more easily exposed to membrane processes without contamination of the membrane by fat. In such processes, the flux over the membrane is generally maintained over time and even higher fluxes can be achieved, which renders the processing and removal of contaminants easier and more economically attractive. It is noted that the absence of fat contamination of a membrane is an indirect measure for the effective encapsulation of the fat by the proteins. The inventive protein composition is bio-based, non-toxic and readily biodegradable. Hence, the inventive protein composition can be used in food and feed applications. A further advantage of hydrolyzed non-functional proteins, in particular of coagulated proteins from Brewer's spent grain, is their improved thermal stability, i.e. these proteins generally remain unchanged after a heat treatment, even up to 90°C; it was observed that higher temperatures do not affect the encapsulation behaviour of these proteins, in particular of hydrolyzed coagulated proteins from Brewer's spent grain. Consequently, the protein composition of the invention can be exposed to pasteurization or sterilization conditions to diminish the microbial activity in the composition.

In the context of this application, the wording "the fat is encapsulated by at least part of the protein" means that the fat is covered with protein in such a way that no fat is exposed. The term "at least part of the protein" means that at least part or all of the protein is used to cover or encapsulate the fat and part of the protein is dissolved and/or dispersed in the solvent or no protein is dissolved and/or dispersed in the solvent. The encapsulation by the protein can be determined using any methods known in the art. Preferably, the confocal scanning laser microscopy with the fluorescent dye Nile Blue. A preferred method of confocal scanning laser microscopy is described by Filippidi et al (2014; doi:10.1002/adfm.201400359).

In one embodiment, the inventive protein composition has an iso-electric point of at most 4. Preferably, the protein composition has an iso-electric point of at most 3.5, and most preferably at most 3, and preferably an iso-electric point of at least 1.5, more preferably at least 2 and most preferably at least 2.5. Not being bound by theory, the iso-electric point is generally lower due to the interaction between fat and the protein of the invention, and hence generally lower than observed for the proteins per se. The iso-electric point can be determined using any conventional methods, e.g. zeta potential measurements.

In one embodiment, the protein composition of the invention comprises particles having a d90 value of at most 50 µm. Preferably, the particles have a d90 value of at most 40 µm, preferably at most 30 µm, more preferably at most 25 µm, even more preferably at most 20 µm, even more preferably at most 15 µm, and most preferably at most 10 µm, and at least 10 nm, preferably at least 200 nm, more preferably at least 500 nm and most preferably at least 1 µm. The particle size distribution, in particular the d90 value, is determined using conventional techniques such as laser diffraction using a Malvern Mastersizer.

In one embodiment, the inventive protein composition comprises at least 80 wt% of the particles in an aqueous suspension or emulsion have particles having a number density or number weighted mean diameter of at least 0.5 µm. Preferably, at least 85 wt% of the particles in an aqueous suspension or emulsion have particles having a number density of at least 0.5 µm, more preferably at least 90 wt%, and most preferably at least 95 wt% of the particles in an aqueous suspension or emulsion have particles having a number density of at least 0.5 µm. The number density is determined using conventional techniques such as laser diffraction.

In one embodiment of the invention, the solubility of the protein composition, in particular the solubility of the fat encapsulated with protein, the fat and/or the protein, in water at 20°C and at a pH of 6 is at least 5 wt%, based on the total weight of the protein composition. Preferably, the solubility of the protein composition in water at 20°C and at a pH of 6 is at least 10 wt%, more preferably the solubility of the protein composition in water at 20°C and at a pH of 6 is at least 15 wt%, even more preferably the solubility of the protein composition in water and at a pH of 6 is at least 20 wt%, and preferably the solubility of the protein composition in water and at a pH of 6 is at most 99 wt%, more preferably the solubility of the protein composition in water and at a pH of 6 is at most 95 wt%, and most preferably the solubility of the protein composition in water and at a pH of 6 is at most 90 wt%, based on the total weight of the protein composition. Solubility can be determined using any conventional methods. Solubility is generally determined by dissolving and/or dispersing the protein composition at the desired pH and temperature, and subsequently centrifugate at a specified g-value (e.g. 4000 rpm for 4 minutes), and measuring the percentage protein left in the supernatant.

The improved solubility of the inventive protein composition renders these compositions to be particularly suitable for acid applications or applications comprising an acidic step. An example of such an application is mayonnaise.

The inventive protein composition comprises fat. The fat can be any fat or lipid known in the art. Fat can already be present together with the protein of the invention, such as fat present in Brewer's spent grain, and/or it can be fat separately isolated. The fat can be animal-derived fat or plant-derived fat. Preferably, the fat used in the invention is plant-based fat. The term "fat" refers to triglycerides, mono- and diglycerides, fatty acids, esters of fatty acids, phospholipids, sterols and waxes. Preferably, the fat is an emulsifiable fat. With the term "emulsifiable fat" is meant the fats that exhibit emulsification properties. Examples of such emulsifiable fats in grain include mono- and disaccharides, fatty acids, esters of fatty acids and phospholipids. Most preferred is fat from Brewer's spent grain.

In one embodiment, the protein composition of the present invention comprises at most 70 wt% fat, more preferably at most 60 wt% fat, even more preferably at most 50 wt% fat and most preferably at most 40 wt% fat, and preferably at least 5 wt% fat, more preferably at least 10 wt% fat, even more preferably at least 15 wt% fat, and most preferably at least 20 wt% fat, based on the total weight of the protein composition. The amount of fat can be determined with methods known in the art including organic solvent extraction. An example of such a technique is the ISO 6492 method.

In one embodiment, the protein composition of the present invention comprises at most 50 wt% fat, more preferably at most 45 wt% fat, even more preferably at most 40 wt% fat and most preferably at most 30 wt% fat, and preferably at least 1 wt% fat, more preferably at least 5 wt% fat, even more preferably at least 10 wt% fat, and most preferably at least 15 wt% fat, based on the total dry weight of the protein composition.

In one embodiment, the protein composition of the present invention comprises at most 80 wt% emulsifiable fat, more preferably at most 70 wt% emulsifiable fat, and most preferably at most 60 wt% emulsifiable fat, and preferably at least 10 wt% emulsifiable fat, more preferably at least 20 wt% emulsifiable fat, even more preferably at least 25 wt% emulsifiable fat, and most preferably at least 30 wt% emulsifiable fat, based on the total weight of the protein composition.

In one embodiment, the protein composition of the present invention comprises at most 80 wt% fat from Brewer's spent, more preferably at most 70 wt% fat from Brewer's spent, and most preferably at most 60 wt% fat from Brewer's spent, and preferably at least 10 wt% fat from Brewer's spent, more preferably at least 20 wt% fat from Brewer's spent, even more preferably at least 25 wt% fat from Brewer's spent, and most preferably at least 30 wt% fat from Brewer's spent, based on the total weight of the protein composition.

In one embodiment, the protein composition of the present invention comprises at most 80 wt% fat from Brewer's spent, more preferably at most 70 wt% fat from Brewer's spent, and most preferably at most 60 wt% fat from Brewer's spent, and preferably at least 10 wt% fat from Brewer's spent, more preferably at least 20 wt% fat from Brewer's spent, even more preferably at least 25 wt% fat from Brewer's spent, and most preferably at least 30 wt% fat from Brewer's spent, based on the total dry weight of the protein composition.

The inventive protein composition comprises a protein, wherein at least 10 wt% of the protein has a molecular weight of at least 3 kDa. The protein can be any protein known in the art. The protein can be an animal-derived protein or a plant-based protein. Preferably, the protein is a plant-based protein. The protein can be a functional protein, a hydrolyzed functional protein or a hydrolyzed non-functional protein. With "functional protein" is meant a protein that is capable of emulsifying oil or fat in water and is capable of stabilizing an oil-in-water emulsion. With "non-functional protein" is meant a protein that is not capable of emulsifying oil or fat in water; such non-functional proteins are generally not soluble in water. Examples of such non-functional proteins include coagulated proteins, such as proteins from brewer's spent grain, and storage proteins such as zein. With "hydrolyzed" is meant the exposure to the process of cutting/shortening the functional and/or the non-functional protein into protein fragments with a smaller molecular weight than the original protein. Hydrolyzation can be achieved using conventional techniques including enzymatic hydrolysis, heat treatment and/or chemical treatment. Preferably, hydrolyzation is achieved by enzymatic hydrolysis. Examples of functional proteins include fava protein and whey protein. Examples of hydrolyzed functional proteins include hydrolyzed soybean protein and hydrolysates of whey protein. Examples of hydrolyzed non-functional proteins includes protein from brewer's spent grain (BSG) and zein. Preferably, the protein is hydrolyzed coagulated protein from BSG.

In one embodiment, the protein composition of the present invention comprises at least 10 wt% protein, based on the total weight of the protein composition. Preferably, the composition comprises at least 15 wt% protein, more preferably at least 20 wt% protein, even more preferably at least 25 wt%, and most preferably at least 30 wt% protein, and preferably at most 80 wt% protein, more preferably at most 75 wt% protein, even more preferably at most 70 wt% protein, even more preferably at most 65 wt% protein and most preferably at most 60 wt% protein, based on the total weight of the protein composition. Various methods have been described in literature to determine the protein content. For the purposes of this application, the Kjeldahl method is used to determine the nitrogen content, which is then converted to protein content. The Kjeldahl is well established and well known to the person skilled in the art. In this application the Kjeldahl method is performed by hydrolyzing a sample using H₂SO₄ at 420°C for 2 hours, during which the proteins will be converted to ammonia. The generated ammonia is distilled off and the amount of nitrogen is measured by titration. The amount of protein is calculated by multiplying the nitrogen content by the conversion factor of 6.25 (nitrogen to protein factor).

In one embodiment, the protein composition of the present invention comprises at least 10 wt% protein, based on the total dry weight of the protein composition. Preferably, the composition comprises at least 15 wt% protein, more preferably at least 20 wt% protein, even more preferably at least 25 wt%, and most preferably at least 30 wt% protein, and preferably at most 90 wt% protein, more preferably at most 80 wt% protein, even more preferably at most 70 wt% protein, even more preferably at most 65 wt% protein and most preferably at most 60 wt% protein, based on the total dry weight of the protein composition.

In one embodiment of the invention, at least 10 wt% of the protein has a molecular weight of at least 3 kDa, based on the total weight of the protein. Preferably, the amount of the protein having a molecular weight of at least 3 kDa is at least 15 wt% protein, more preferably at least 20 wt% protein, even more preferably at least 25 wt%, and most preferably at least 30 wt% protein, and preferably at most 100 wt% protein, more preferably at most 99 wt% protein, even more preferably at most 95 wt% protein, even more preferably at most 90 wt% protein and most preferably at most 80 wt% protein, based on the total weight of the protein.

In one embodiment, the molecular weight of the protein is at most 700 kDa, more preferably at most 600 kDa, and most preferably at most 500 kDa. The molecular weight distribution of the protein can be determined using conventional analytical techniques, e.g. based on size exclusion chromatography. A preferred method is a method based on Phenomenex POLYSEP.

In one embodiment of the invention, the amount of the protein having a molecular weight of at most 3 kDa is at most 80 wt%, based on the total weight of the protein. Preferably, the amount of the protein having a molecular weight of at most 3 kDa is at most 60 wt% protein, more preferably at most 55 wt% protein, even more preferably at most 50 wt%, and most preferably at most 40 wt% protein, and preferably at least 0.01 wt% protein, more preferably at least 0.05 wt% protein, even more preferably at least 0.1 wt% protein, even more preferably at least 0.5 wt% protein and most preferably at least 1 wt% protein, based on the total weight of the protein. It is advantageous to reduce the amount of proteins having a molecular weight of at most 3 kDa as these short chain proteins generally have less or no affinity to the fat, and do not exhibit good emulsifying properties.

In one embodiment of the invention, the amount of the coagulated protein is at most 20 wt%, based on the total weight of the protein. Preferably, the amount of the coagulated protein is at most 15 wt% coagulated protein, more preferably at most 10 wt% coagulated protein, even more preferably at most 5 wt% coagulated protein, and most preferably at most 1 wt% coagulated protein, and preferably at least 0.001 wt% coagulated protein, more preferably at least 0.005 wt% coagulated protein, even more preferably at least 0.01 wt% coagulated protein, even more preferably at least 0.05 wt% coagulated protein and most preferably at least 0.1 wt% coagulated protein, based on the total weight of the protein. In a preferred embodiment, the protein composition comprises no coagulated protein. Coagulated protein is generally not soluble and can generally be removed effectively. The presence of coagulated protein can be determined using conventional analytical techniques such as centrifugation of coagulated protein from emulsions with droplet size which is dependent of protein concentration (as emulsifier).

In one embodiment of the invention, the weight ratio of the fat and the protein, is between 0.1 and 10. Preferably, the weight ratio between fat and protein is at least 0.2, more preferably at least 0.3, even more preferably at least 0.4, and most preferably at least 0.5, and preferably at most 8, more preferably at most 5, even more preferably at most 4, and most preferably at most 2.

In one embodiment, the protein composition of the present invention comprises water. Preferably, the composition comprises at most 99 wt% water, more preferably at most 95 wt% water, and most preferably at most 90 wt% water, and preferably at least 1 wt% water, more preferably at least 2 wt% water, even more preferably at least 5 wt% water, and most preferably at least 10 wt% water, based on the total weight of the protein composition. The composition of the invention can be in any form known in the art. The protein composition may be liquid or may be solid. The amount of water or other solvent present in the inventive composition generally determines whether the composition is an emulsion, a paste or powder.

In one embodiment, the protein composition of the present invention comprises less than 2 wt% insoluble fiber, based on the total weight of the protein composition. Preferably, the composition comprises at most 1.5 wt% insoluble fiber, more preferably at most 1.2 wt% insoluble fiber, and most preferably at most 1 wt% insoluble fiber, and preferably at least 0.001 wt% insoluble fiber, more preferably at least 0.01 wt% insoluble fiber, even more preferably at least 0.05 wt% insoluble fiber, and most preferably at least 0.1 wt% insoluble fiber, based on the total weight of the protein composition. The insoluble fiber can be determined using conventional analytical techniques. An example of such a technique is the ICUMSA GS2-19 method.

The remaining part of the protein composition may be comprised of other components commonly used in protein compositions, such as excipients like preservatives, pigments or dyes, etc. With the protein composition, water, the other components add up to 100 wt% of the total weight of the protein composition.

The invention moreover pertains to an emulsion comprising water, oil and the protein composition according to the invention. The emulsion can be any emulsion known in the art. The emulsion of the invention comprises at least two liquids and can be an oil-in-water emulsion. Preferably, the emulsion is an oil-in-water emulsion. The inventive protein composition generally emulsifies and stabilizes the oil droplets in the water matrix. Also the amount of oil (or water) used can be high (e.g. above 50 wt% oil in the emulsion). Due to the pH stability of the protein composition, emulsions can be prepared at different pH values, including emulsions where lowering to a low pH (e.g. below a pH of 5) and subsequent increase of pH (above 6). As the inventive protein composition is bio-based, non-toxic and biodegradable, the inventive composition can be used in food-grade applications, such as dressings, sauces and mayonnaise.

The oil suitable in the emulsion of the invention is any oil or hydrophobic liquid known in the art. Examples of such oils include hydrocarbon oils such as mineral oil fractions comprising linear mineral oils (n-paraffins), branched mineral oils (iso-paraffinic) and/or cyclic mineral oils (naphthenic oils); polyisobutylenes (PIB), phosphate esters such as trioctyl phosphate; polyalkylbenzens such as heavy alkylates, dodecyl benzene and other alkylarenes; esters of aliphatic monocarboxylic acids; linear or branched mono unsaturated hydrocarbons such as linear or branched alkanes containing 8 to 25 carbon atoms and linear or branched alkenes containing 8 to 25 carbon atoms; natural flavor and/or fragrance oils such as essential oils, jojoba oil and flavor oils derived from citrus peel; and natural oils such as palm oil, soybean oil, olive oil, sunflower oil, rapeseed oil and castor oil.

In one embodiment of an oil-in-water emulsion, the emulsion may comprise the oil in an amount of at most 70 % by weight (wt%), based on the total weight of the emulsion. Preferably, the oil is present in an amount of at most 60 wt%, more preferably at most 50 wt%, even more preferably at most 40 wt% and most preferably at most 30 wt%, and preferably at least 1 wt%, more preferably at least 2 wt%, even more preferably at least 5 wt% and most preferably at least 10 wt%, based on the total weight of the emulsion.

In one embodiment of an oil-in-water emulsion, the emulsion may comprise water in an amount of at least 30 % by weight (wt%), based on the total weight of the emulsion. Preferably, water is present in an amount of at least 40 wt%, more preferably at least 50 wt%, even more preferably at least 60 wt% and most preferably at least 70 wt%, and preferably at most 99 wt%, more preferably at most 98 wt%, even more preferably at most 95 wt% and most preferably at most 90 wt%, based on the total weight of the emulsion.

The protein composition of the invention can be chosen according to need in amounts as desired. The emulsion of the invention, preferably the oil-in-water emulsion, may comprise the protein composition in an amount of at most 10 % by weight (wt%), based on the total weight of the emulsion. Preferably, the protein composition is present in an amount of at most 8 wt%, more preferably at most 5 wt%, even more preferably at most 2 wt% and most preferably at most 1 wt%, and preferably at least 0.01 wt%, more preferably at least 0.05 wt%, even more preferably at least 0.1 wt% and most preferably at least 0.2 wt%, based on the total weight of the emulsion.

The remaining part of the emulsion may be comprised of other components commonly used in emulsions such as salts, preservatives, pigments and dyes, fragrances or flavoring agents, etc. With the oil, water and the protein composition the other components add up to 100 wt% of the total weight of the emulsion.

The emulsions of the invention can be used in food application, paints, construction applications, textiles e.g. fiber treatment, leather lubrication, household care compositions, softening, fabric care in laundry applications, healthcare applications, release agents, water-based coatings and personal care or cosmetic applications. The cosmetic applications include those that are intended to be placed in contact with portions of the human body (skin, hair, nails, mucosa, etc.) or with teeth and the mucous membranes of the oral cavity with a view exclusively or mainly to cleaning them, perfuming them, changing their appearance, protecting them, keeping them in good condition or modifying odors, and skin care, sun care, hair care or nail care applications. Consequently, the present invention further pertains to emulsions of the invention, in particular oil-in-water emulsions, for use in creams, ointments, unguents, gels, pastes, liniments, foams, transdermal patches, lotions and topical solutions. The invention further pertains to a cosmetic composition comprising a protein composition according to the invention and a cosmetic excipient. The cosmetic excipient can be any excipient or additive used in cosmetic products.

The invention further pertains to a dispersion comprising the protein composition of the invention, a solvent, and solid particles. The protein composition serves as a dispersant enabling solid particles to remain in the solvent rather than settling on the bottom. The inventors found that the inventive protein composition can form and stabilize dispersions of solids, in particular inorganic solid particles.

The solid particles or solids may be any solid particle known in the art and which can be suitably used in dispersions. The solid particles of the invention may be selected from polymeric particles, oxide and/or hydroxide particles, pigments and fillers.

Examples of polymeric particles include polyolefins such as polystyrene, polyethylene and polypropylene; polyesters such as polyacrylate and polymethyl methacrylate. Further examples of monomers of suitable polymers have been described above.

Examples of oxides and/or hydroxides particles include oxides and hydroxides of aluminum, silicium, boron, sodium, potassium, calcium, iron, nickel, cobalt, titanium, zirconium, cerium, chromium, zinc, tin and tungsten. Preferably, the oxide and/or hydroxides particles are oxides and/or hydroxides selected from aluminum, silicium, calcium, titanium and iron.

Examples of pigments include metal-based pigments, inorganic pigments and biological and organic pigments. Examples of metal-based pigments include cadmium pigments such as cadmium yellow cadmium green, cadmium orange, cadmium sulfoselenide and cadmium red; cobalt pigments such as cobalt violet, cobalt blue, aureolin and cerulean blue; chromium pigments such as chrome yellow and chrome green (viridian); copper pigments such as azurite, Han purple, Han blue and Egyptian blue; iron oxide pigments such as sanguine, caput mortuum, red ochre, Venetian red and Prussian blue; lead pigments such as lead white, Naples yellow, red lead and lead-tin-yellow; manganese pigments such as manganese violet and YlnMn blue; mercury pigments such as vermillion; titanium pigments such as titanium yellow, titanium beige, titanium white and titanium black; and zinc pigments such zinc white, zinc ferrite and zinc yellow. Examples of (non-metal-based) inorganic pigments include carbon pigments such as carbon black and ivory black and clay earth pigments such as yellow ochre, raw sienna, burnt sienna, raw umber and burnt umber; and ultramarine pigments such as ultramarine and ultramarine green shade. Examples of biological pigments include alizarin, alizarin crimson, gamboge, cochinal red, rose madder, indigo, indian yellow and Tyrian purple. Examples of organic pigments include quinacridone, magenta, phthalo green, phthalo blue, pigment red 170 and diarylide yellow.

Examples of fillers include calcium carbonates such as ground calcium carbonate (GCC) and precipitated calcium carbonate (PCC), kaolin, carbon black, talc, bentonite, hydrotalcite and hydrotalcite-like clays, diatomite, limestone, titanium dioxide, wood flour, saw dust, calcium sulphate, aluminum trihydrate, aluminum silicate and silica.

In one embodiment, the dispersion may comprise the solid particles in an amount of at most 60 % by weight (wt%), based on the total weight of the dispersion. Preferably, the solid particles are present in an amount of at most 50 wt%, more preferably at most 40 wt%, even more preferably at most 30 wt% and most preferably at most 25 wt%, and preferably at least 1 wt%, more preferably at least 2 wt%, even more preferably at least 5 wt% and most preferably at least 10 wt%, based on the total weight of the composition.

In one embodiment of the invention, the solid particles have a d90 value of at most 100 µm, preferably at most 50 µm, more preferably at most 20 µm, even more preferably at most 10 µm, even more preferably at most 5 µm, and most preferably at most 1 µm, and at least 10 nm, preferably at least 20 nm, more preferably at least 50 nm and most preferably at least 100 nm.

In one embodiment of the invention, the solid particles have a d99 value of at most 100 µm, preferably at most 50 µm, more preferably at most 20 µm, even more preferably at most 10 µm, even more preferably at most 5 µm, and most preferably at most 1 µm, and at least 10 nm, preferably at least 20 nm, more preferably at least 50 nm and most preferably at least 100 nm.

The dispersion of the invention further comprises a solvent. The solvent can be any solvent known in the art and suitable for use in dispersions. Examples of solvents include water; alcohols such as ethanol and isopropanol; alkanes such as pentane and hexane; ketones such as methyl ethyl ketone (MEK), acetone and methyl propyl ketone; and aromatic solvents such as toluene and benzene. In a preferred embodiment, the dispersion comprises water as solvent. Especially, the solvents other than water increase the amount of volatile organic compounds (VOC). Therefore, dispersions of the invention are preferred that only comprise water as solvent.

In one embodiment, the dispersion may comprise water (or a solvent) in an amount of at most 90 % by weight (wt%), based on the total weight of the dispersion. Preferably, water is present in an amount of at most 80 wt%, more preferably at most 70 wt%, even more preferably at most 60 wt% and most preferably at most 50 wt%, and preferably at least 1 wt%, more preferably at least 2 wt%, even more preferably at least 5 wt% and most preferably at least 10 wt%, based on the total weight of the dispersion.

The remaining part of the dispersion may be comprised of other components commonly used in dispersions. With the solid particles, the solvent or water and the protein composition, the other components add up to 100 wt% of the total weight of the dispersion.

The invention further pertains to a process for preparing a protein composition according to the invention comprising the steps of:
(a) optionally contacting a hydrolysable protein with an enzyme capable of hydrolyzing said hydrolysable protein to form a functional protein of which at least 10 wt% has a molecular weight of at least 3 kDa;
(b) contacting fat and the functional protein of which at least 10 wt% of the protein has a molecular weight of at least 3 kDa to a temperature above 30°C to obtain encapsulated fat;
(c) optionally subjecting the encapsulated fat-containing suspension to ultrafiltration with a MWCO of at least 50 kDa to separate at least a portion of small molecules and salts from the suspension to form a retentate comprising the protein composition;
(d) optionally separating the fat and the protein; and
(e) optionally removing water from the retentate.

In optional step (a) of the inventive process a hydrolysable protein is contacted with an enzyme to hydrolyze the protein to obtain a functional protein of which at least 10 wt% has a molecular weight of at least 3 kDa. The functional protein generally is an aqueous dispersion of the hydrolysable protein and/or a hydrolyzed portion of the hydrolysable protein. The conditions of the enzymatic hydrolysis can be chosen to selectively obtain a larger fraction of proteins having a molecular weight of at least 3 kDa. Also, the enzyme can be selected to optimize for this protein fraction. The enzyme can be any enzyme known in the art and suitable for hydrolyzing proteins. Examples of such enzymes include serine protease such as Subtilisin; aspartic protease such as Pepsin-like protease and Rennin-like protease; cysteine/thiol protease such as papain; and metalloprotease such as Neutrase^{®}. The skilled person will understand the optimal conditions for the enzymatic hydrolysis. It is noted that the conditions can also be chosen less optimal to enable a better selectivity towards the functional proteins of which at least 10 wt% have molecular weights of at least 3 kDa.

The functional protein obtained in step (a) can be contacted with fat in step (b) to obtain the encapsulated fat. Fat may be added to the functional protein and/or the fat was already present during the hydrolysis step (a). The suspension or emulsion is maintained at a temperature above 30°C. Preferably, the temperature of the suspension is at least 35°C, more preferably at least 40°C, more preferably at least 45°C and most preferably at most 50°C, and preferably at most 100°C, more preferably at most 95°C, and most preferably at most 90°C.

In optional step (c) of the process, the suspension is subsequently subjected to membrane filtration, in particular ultrafiltration with a MWCO (molecular weight cut off) of at least 50 kDa. Preferably, the MWCO is at least 75 kDa, more preferably at least 100 kDa, even more preferably at least 150 kDa and most preferably at least 200 kDa, and preferably at most 500 kDa, more preferably at most 450 kDa and most preferably at most 400 kDa. Depending on the conditions of ultrafiltration (flow rate, temperature, MWCO and type of membrane), minerals or salts and low molecular weight proteins are effectively separated from the protein composition and removed through the permeate. The protein composition is maintained in the retentate.

In optional step (d), the fat and the protein that generally interact are separated through conventional means. Such conventional means are generally known to the skilled person and may include a pH and/or temperature change and/or a chemical treatment. Further processing may be required to separate the free fat and protein. Further processing may include precipitation, decantation, centrifugation and (ultra)filtration.

In the optional step (e), water may be at least partially removed from the retentate comprising the protein composition. Water removal can be performed using conventional techniques including membrane separation such as (reverse) osmosis and evaporation techniques such as fluidized bed drying, spray drying and evaporation through heating the retentate. In this way, an aqueous suspension comprising protein composition can be obtained. When the protein composition of the invention is dried further a powder can be obtained.

The invention further pertains to a protein composition obtainable by the process of the invention as described above. The protein composition may comprise fat and protein, preferably fat encapsulated by protein. Alternatively, the product obtained with the inventive process may only comprise protein, while the fat is removed. The molecular weight distribution of the proteins is generally different from the original protein as the proteins are obtained that selectively interact with the fat, while the proteins that do not interact with the fat can be removed.

The protein composition of the invention can be used in a wide range of applications. The invention pertains to the use of the protein composition in food applications, paints, construction applications, textiles e.g. fiber treatment, leather lubrication, household care compositions, softening, fabric care in laundry applications, healthcare applications, release agents, water-based coatings, personal care or cosmetic applications, emulsion polymerizations, carpeting, automobile parts, window frames, kitchen worktops, container closures, lunch boxes, closures, medical devices, household articles, food containers, dishwashers, outdoor furniture, blow-molded bottles, disposable non-woven fabrics, cables and wires, packaging, coil coating applications, can coatings, car refinish, mining, oil drilling, fuel additive and automotive applications. Each of these uses is separately contemplated and is meant to be explicitly and individually disclosed.

The invention is exemplified in the following Examples.

### Examples

### Example 1

A 305 kg of isolated protein of Brewer's spent grain (26.9 wt% of dry matter; 43.8 wt% protein and 17.7 wt% fat (Berntrop)) is diluted with 732 kg demineralized water. 160 ml of a Neutrase^{®} solution was added to the suspension. The suspension was set to a pH of 6 using a 5 M NaOH solution and maintained at a pH of 6 for 4 hours. Subsequently, the resulting suspension was heated to 90°C for 10 minutes and cooled to 20°C. The resulting suspension is dewatered using a filter press at a temperature of 50°C. The filtrate is subsequently centrifuged at 50°C using a disc-stack centrifuge. The resulting liquid was led over an ultrafiltration unit with an MWCO of 300 kDa. The first retentate is collected and analyzed. The permeate was led over an ultrafiltration unit with an MWCO of 1 kDa. The second retentate was collected and analyzed.

The first retentate contained 34.8 wt% protein (Kjeldahl) and 43.5 wt% fat (Berntrop). The protein contained 32.6 wt% of proteins having a molecular weight of at least 3 kDa using size exclusion chromatography (Phenomenex POLYSEP). The iso-electric point is 2.8. The d90 value was determined at 6.7 µm as determined using laser diffraction.

The second retentate contained 92.8 wt% protein and 0 wt% fat (Berntrop). The protein contained 10.6 wt% of proteins having a molecular weight of at least 3 kDa. The d90 value was determined at 140 µm. It appears that the proteins in the second retentate that have passed through the 300 kDa membrane form larger sized protein coagulates.

### Kjeldahl method

The Kjeldahl method was performed by hydrolyzing a sample using H₂SO₄ at 420°C for 2 hours, during which the proteins will be converted to ammonia. The generated ammonia is distilled off and the amount of nitrogen is measured by titration. The amount of protein is calculated by multiplying the nitrogen content by the conversion factor of 6.25 (nitrogen to protein factor).

### Example 2: Mayonnaise

A model mayonnaise comprising the protein composition of Example 1 (first retentate) was prepared with the following composition (in g/100g):

| | |
|---|---|
| First retentate | 11 |
| Sunflower oil | 75 |
| NaCl | 1.2 |
| EDTA | 0.008 |
| Acetic acid | add until pH reaches 3.8 |
| Water | make up to 100 gram |

### Preparation of an aqueous phase:

- disperse the protein composition of Example 1 (first retentate) in demineralized water, stir with magnetic stirrer
- raise pH to 6.5 using 1 M NaOH (to dissolve pectin)
- add NaCl and ethylenediaminetetraacetic acid (to prevent lipid oxidation)
- lower pH to 3.8 with concentrated acetic acid

### Preparation of the emulsion:

- slowly add sunflower oil to aqueous phase while stirring with a high shear mixer (Silverson L5M-A, fine emulsor screen, 3500 rpm)
- after all oil is added, continue stirring at 8000 rpm for 4 minutes
- store emulsion at 5 °C

The resulting model mayonnaise had a light and smooth appearance and was stable as was shown in microscopic images of the mayonnaise where small stable particles were observed. It is noted that the model mayonnaise and any mayonnaise containing the protein composition instead of egg yolk renders the mayonnaise fully plant-based and is suitable for vegan consumers.

A similar mayonnaise is created with the second retentate of Example 1. The mayonnaise was not stable and eventually two layers were formed. Microscopy showed some emulsification with small and larger droplets and coagulated areas. The protein composition of the second retentate cannot stabilize oil droplets in mayonnaise.

## Claims

1. A protein composition comprising fat and a protein wherein the fat is encapsulated by at least part of the protein and at least 10 wt% of the protein has a molecular weight of at least 3 kDa, and the amount of protein is at least 5 wt%, based on the total weight of the composition.

2. Protein composition according to claim 1 having an iso-electric point of at most 4.

3. Protein composition according to any one of claims 1 and 2 comprising particles having a d90 value of at most 50 µm.

4. Protein composition according to any one of the preceding claims wherein at least 80 wt% of the particles in an aqueous suspension or emulsion have particles having a number density of at least 0.5 µm.

5. Protein composition according to any one of the preceding claims wherein the protein is a hydrolyzed non-functional protein, preferably the non-functional protein is a coagulated protein and/or a storage protein.

6. Protein composition according to claim 5 wherein the non-functional protein is coagulated protein from brewer's spent grain.

7. Protein composition according to any one of the preceding claims wherein the composition comprises at least 10 wt% of protein, based on the total weight of the composition.

8. Protein composition according to any one of the preceding claims wherein the weight ratio of the fat and the protein, is between 0.1 and 10.

9. Protein composition according to any one of the preceding claims wherein the fat is an emulsifier fat.

10. Cosmetic composition comprising a protein composition according to any one of the preceding claims and a cosmetic excipient.

11. An emulsion comprising water, oil and the protein composition according to any one of claim 1 to 9.

12. A dispersion comprising a solvent, solid particles and the protein composition according to any one of claim 1 to 9.

13. Use of the protein composition according to any one of claims 1 to 9 in a food product.

14. Use of the protein composition according to any one of claims 1 to 9 in a cosmetic product.
